# EUROPÄISCHE PATENTANMELDUNG

(11) **EP 1 228 775 A2**
(43) Veröffentlichungstag der Anmeldung: **07.08.2002**
(21) Anmeldenummer: 02001572.3
(22) Anmeldetag: 23.01.2002
(51) Int. Cl.: A61L 31/02, A61L 31/08

(54) **Osteosynthesehilfsmittel aus einer Stahl-, Cobalt- und/oder Titanlegierung**

(30) Priorität: 03.02.2001 DE 20101917 U
(71) Anmelder: Stryker Trauma GmbH, 24232 Schönkirchen (DE)
(72) Erfinder: Speitling, Andreas, Dr., 24149 Kiel (DE); Oldenburg, Geert von, 24105 Kiel (DE)
(74) Vertreter: Patentanwälte Hauck, Graalfs, Wehnert, Döring, Siemons

(57) **Zusammenfassung**

Osteosynthesehilfsmittel aus einer Stahl-, Cobalt- und/oder Titanlegierung, wobei in einem Abschnitt, der im eingesetzten Zustand in Berührung mit Knochengewebe oder mit Metall steht, die Oberfläche modifiziert ist derart, daß Korrosion und Abrieb verringert bzw. verhindert werden.

## Beschreibung

Die Erfindung betrifft ein Osteosynthesehilfsmittel aus einer Stahl-, Cobalt- und/oder Titanlegierung.

Osteosynthesehilfsmittel in Form von Implantaten der Traumatologie und Endoprothetik sind hinreichend bekannt, ebenso wie Fixateur Externe. Die eingesetzten Implantate sind im Körper des Patienten Korrosion, Reibung und Verschleiß ausgesetzt. Die Korrosions- und Verschleißschäden treten dabei insbesondere zwischen einzelnen Implantat-Komponenten auf, die in ihren Kontaktbereichen Mikrobewegungen gegeneinander ausführen können. Ebenfalls treten Korrosions- und Verschleißschäden im Kontaktbereich zwischen Implantat und Knochen durch Mikrobewegungen und aufgrund von an dem Knochen angreifenden Belastungen auf.

Diese Schäden an den eingesetzten Osteosynthesehilfsmitteln führen bei der Behandlung des Patienten dazu, daß metallische Ionen aus den Implantatbauteilen ins angrenzende Gewebe freigesetzt werden. Diese Ionen können teilweise für den Körper unverträgliche Eigenschaften besitzen und somit zu toxischen Körperreaktionen, Entzündungen, Knochenrückbildung, Heilungsstörung und ähnlichem führen. Ebenfalls können durch diese Funktionsstörungen des Implantats, Korrosions- und Verschleißschäden auftreten, die beispielsweise zu einer Reibkraftzunahme und Verklemmung zwischen zwei beweglichen Bauteilen des Implantats führen. Korrosions- und Verschleißschäden führen auch zur Verringerung der statischen und im besonderen Maße der dynamischen Festigkeit.

Der Erfindung liegt die Aufgabe zugrunde ein Osteosynthesehilfsmittel bereitzustellen, bei dem auftretende Korrosion, Abrieb und Verschleiß verringert bzw. verhindert wird. Ebenso liegt der Erfindung die Aufgabe zugrunde, die Funktionalität eines Osteosynthesehilfsmittels mit Hilfe von Reibkräften zu erhöhen.

Erfindungsgemäß wird die Aufgabe durch ein Osteosynthesehilfsmittel mit den Merkmalen aus Anspruch 1 gelöst. Das Osteosynthesehilfsmittel besitzt einen Abschnitt, der im eingesetzten Zustand in Berührung mit Knochengewebe oder Metall steht. Erfindungsgemäß ist die Oberfläche mindestens in diesem Bereich derart modifiziert, daß Korrosion und Abrieb verringert bzw. verhindert werden. Der Erfindung liegt die Erkenntnis zugrunde, daß bereits durch eine Modifikation der Implantatoberfläche Korrosion und Abrieb sich verringern bzw. verhindern lassen. Auch liegt der Erfindung die Erkenntnis zugrunde, daß eine Oberflächenmodifikation nicht für das gesamte Osteosynthesehilfsmittel erfolgen muß, sondern bereits die Oberflächenmodifikation in dem Abschnitt ausreicht, der im eingesetzten Zustand Abrieb und Korrosion ausgesetzt ist.

In einer ersten bevorzugten Ausgestaltung der Oberflächenmodifikation wird der Abschnitt des Osteosynthesehilfsmittels durch eine Beschichtung modifiziert. In einer Ausgestaltung wird die Beschichtung durch PVD (physical vapour deposition) oder CVD (chemical vapour deposition) aufgebracht. Bevorzugt sind hier DLC-Schichten (Diamond like carbon) und deren Untergruppen, insbesondere Schichten vom Typ MeC/CH, also beispielsweise Schichten mit den Bestandteilen Tantal, Kohlenstoff und Wasserstoff (TaC/CH).

In einer weiteren Ausgestaltung des erfindungsgemäßen Osteosynthesehilfsmittels ist der modifizierte Abschnitt durch ein Diffusionsverfahren gehärtet. Das Diffusionsverfahren kann zusätzlich zu der Beschichtung verwendet werden. Bevorzugt wird durch das Diffusionsverfahren bei einer Titanlegierung Stickstoff oder Sauerstoff, z. B. ODH-Verfahren, und bei einer Stahllegierung Kohlenstoff, z. B. Kolsterieren, in den zu härtenden Abschnitt eingebracht.

Eine weitere Ausgestaltung der Oberflächenmodifikation bei dem erfindungsgemäßen Osteosynthesehilfsmittel sieht die Verwendung eines Anodisierverfahrens vor. Das Anodisierverfahren kann insbesondere zu einer Anodisation vom Typ II oder vom Typ III durch eine elektrolytische Behandlung führen. Üblicherweise wird die elektrolytische Behandlung in speziellen dafür vorgesehenen Bädern durchgeführt. Ein solches Verfahren ist beispielsweise das als Dotize bezeichnete Verfahren der Firma DOT. Das Anodisierverfahren kann ebenfalls in Kombination mit anderen oben genannten Verfahren eingesetzt werden, um eine ausreichende Oberflächenmodifikation zu erzielen.

In einer anderen Ausgestaltung des erfindungsgemäßen Osteosynthesehilfsmittels ist der Abschnitt durch spanende oder spanlose, formgebende Bearbeitungsverfahren modifiziert. Solche Bearbeitungsverfahren können die Verfahrensschritte des Glas- und/oder Sandstrahlens aufweisen. Auch ist es möglich, verschiedene Strahlvorgänge mit Glas-, Metall- oder Keramikkugeln unterschiedlicher Größe und mit unterschiedlichen Strahlparametern durchzuführen. Auch kann das Bearbeitungsverfahren die Arbeitsschritte des Gleitschleifens aufweisen. Ferner ist es möglich, durch mechanisches Polieren und/oder Elektropolieren die Oberfläche in besonderem Maße zu glätten.

In einer besonders bevorzugten Weiterführung der Erfindung ist als Osteosynthesehilfsmittel ein intramedullärer Nagel vorgesehen, der im eingesetzten Zustand mit Hilfe von einer oder mehreren Verriegelungsschrauben im Knochen fixiert ist. Erfindungsgemäß ist der intramedulläre Nagel und/oder die Verriegelungsschraube in ihrem jeweiligen Kontaktbereich mit einer modifizierten Oberfläche versehen.

Hierdurch wird erzielt, daß bei einem Aneinanderreiben von intramedullärem Nagel und Verriegelungsschraube es nicht zu Korrosion und Verschleiß der beiden Komponenten kommt.

In einer weiteren bevorzugten Ausgestaltung ist als Osteosynthesehilfsmittel ein intramedullärer Nagel vorgesehen, der im eingesetzten Zustand eine Schenkelhalsschraube aufnimmt. Erfindungsgemäß sind der intramedulläre Nagel und/oder die Schenkelhalsschraube in ihrem jeweiligen Kontaktbereich mit einer modifizierten Oberfläche versehen.

In einer weiteren Ausgestaltung des erfindungsgemäßen Osteosynthesehilfsmittels ist als Implantat ein intramedullärer Nagel vorgesehen, dessen Oberfläche im Kontaktbereich mit dem Knochen modifiziert ist.

In einer weiteren Ausgestaltung ist als Osteosynthesehilfsmittel eine Hüftplatte vorgesehen, die mit einer Schenkelhalsschraube und/oder Verriegelungsschrauben am Knochen gehalten ist, wobei die Hüftplatte und/oder die Schenkelhalsschraube sowie die Verriegelungsschraube in ihren jeweiligen Kontaktbereichen eine modifizierte Oberfläche ausweisen.

Die Hüftplatte kann zusätzlich zu der oben genannten modifizierten Oberfläche eine modifizierte Oberfläche in dem mit Knochengewebe in Kontakt stehenden Bereich aufweisen.

Die erfindungsgemäße Aufgabe wird ebenfalls durch ein Osteosynthesehilfsmittel aus einer Stahl-, Cobalt- und/oder Titanlegierung gelöst, das die Merkmale aus Anspruch 20 aufweist.

Als Osteosynthesehilfsmittel ist hier ein Fixateur Externe mit mindestens einem Knochen-Pin, einer Konstruktionsstange und einer Klemmvorrichtung zur Klemmung von Knochen-Pin und Konstruktionsstange vorgesehen, wobei im Klemmbereich eine modifizierte Oberfläche vorgesehen ist derart, daß die Reibung zwischen Klemmeinrichtung und Knochen-Pin sowie Konstruktionsstange erhöht ist. Hierdurch wird erzielt, daß durch die Klemmeinrichtung die Konstruktionsstange sowie der Knochen-Pin sicher gehalten sind und nicht die Gefahr eines Durchrutschens besteht.

Vorteilhafte Ausführungsformen des erfindungsgemäßen Osteosynthesehilfsmittels werden nachfolgend mit Bezug auf die Figuren näher erläutert.

Es zeigt:
- Fig. 1: einen intramedullären Nagel mit einer Schenkelhalsschraube und Verriegelungsschrauben,
- Fig. 2: eine schematische Ansicht einer Hüftplatte mit Verriegelungsschrauben und einer Schenkelhalsschraube,
- Fig. 3: eine schematische Ansicht eines intramedullären Nagels mit einer Schenkelhalsschraube und
- Fig. 4: einen Fixateur Externe mit einem Knochen-Pin und einer Konstruktionsstange.

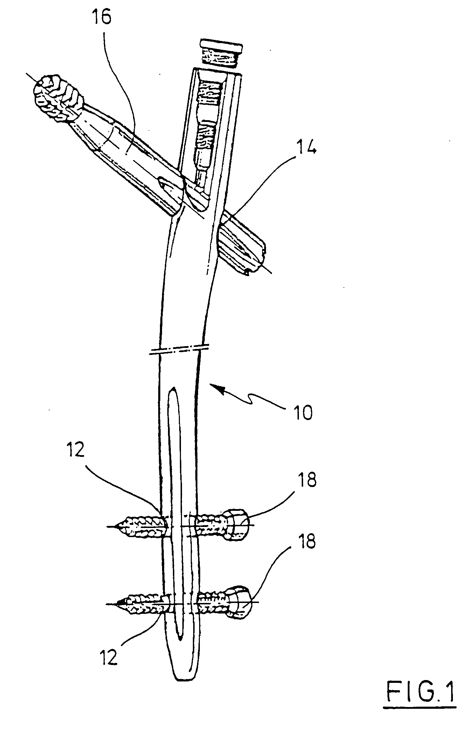

Fig. 1 zeigt eine schematische Ansicht eines intramedullären Nagels 10. Der intramedulläre Nagel besitzt an seinem distalen Ende zwei quer zur Längsachse sich erstreckende Bohrungen 12. An seinem proximalen Ende besitzt der intramedulläre Nagel 10 eine quer zur Längsachse verlaufende Bohrung 14. In die Bohrung 14 eingesetzt ist eine Schenkelhalsschraube 16. Der Kontaktbereich zwischen Bohrung 14 und Schenkelhalsschraube 16 besitzt eine modifizierte Oberfläche, die zu einer Verminderung der Schäden aufgrund einer Bewegung von Schenkelhalsschraube gegenüber dem intramedullären Nagel führt. Die Oberflächenmodifikation ist sowohl an der Schenkelhalsschraube 16 als auch auf der Innenfläche der Bohrung 14 vorgesehen, jedoch kann diese auch ausschließlich an dem intramedullären Nagel oder ausschließlich an der Schenkelhalsschraube vorgenommen sein.

Die Bohrung 12 für die Verriegelungsschrauben 18 sind ebenfalls mit einer modifizierten Oberfläche ausgestattet, um einen Verschleiß oder Abrieb von Material zu verhindern. Auch hierbei können ebenfalls die Verriegelungsschrauben 18 mit einer modifizierten Oberfläche ausgestattet sein.

Für die Oberflächenmodifikation kann die vorteilhafte Wirkung der Erfindung erzielt werden, sowohl durch eine Oberflächenbeschichtung als auch durch eine Oberflächenhärtung. Beide Oberflächenmodifikationen für sich genommen oder in der Kombination sorgen dafür, daß insbesondere auch bei einer längeren Verweildauer des intramedullären Nagels im Knochen keine metallischen Ionen in den Körper freigesetzt werden und der verwendete Nagel nicht an seiner statischen und vor allem nicht an seiner dynamischen Festigkeit verliert.

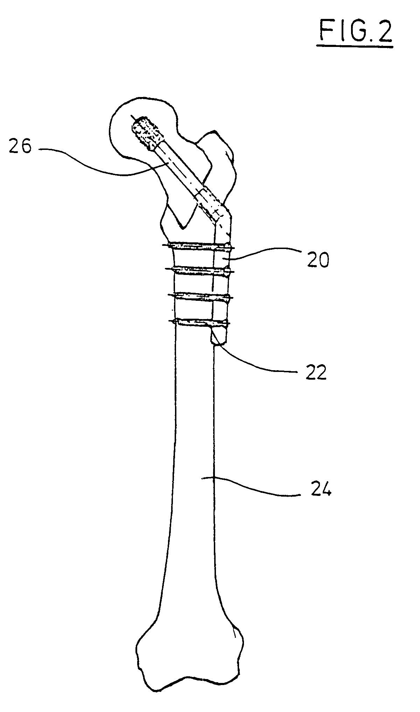

Fig. 2 zeigt die schematische Ansicht einer Hüftplatte 20. Die Hüftplatte 20 besitzt Bohrungen zur Aufnahme von Verriegelungsschrauben 22. Im eingesetzten Zustand liegt die Hüftplatte 20 an dem Knochen 24 an. Die Hüftplatte dient zusätzlich zur Befestigung einer Schenkelhalsschraube 26, die einen Schenkelhalsbruch fixiert. Auch bei der Verwendung der Hüftplatte 20 entsteht durch Bewegung und Belastung des Knochens 24 Reibung zwischen der Schenkelhalsschraube 26, der Verriegelungsschraube 22 und dem Knochenmaterial 24 gegenüber der Hüftplatte 20. So besteht ein Flächenkontakt von der Schenkelhalsschraube zu der Hüftplatte, wobei beide aufeinander gleiten können, was zum Auftreten von Reibung, Verschleiß und Korrosion führt. Ebenso führen die Verriegelungsschrauben 22 in den Bohrungen der Hüftplatte 20 Mikrobewegungen aus, die zum Auftreten von Verschleiß und Korrosion führen. Hinzu kommen die Mikrobewegungen zwischen der Hüftplatte und dem Knochen, die möglicherweise zur Freisetzung von Metallionen führen. Durch die Oberflächenmodifikation der Hüftplatte kann dies wirkungsvoll vermindert bzw. verhindert werden.

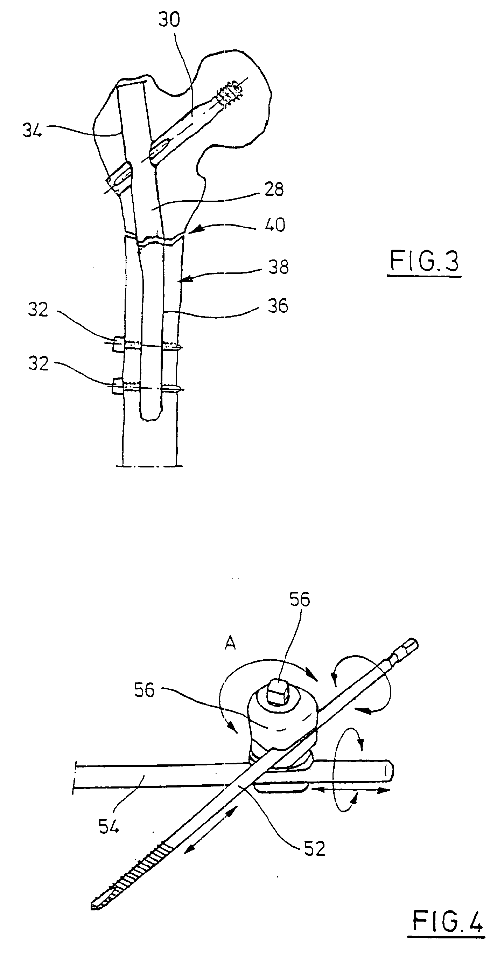

Fig. 3 zeigt eine schematische Ansicht eines intramedullären Nagels 28 mit einer Schenkelhalsschraube 30 und zwei Verriegelungsschrauben 32. Der intramedulläre Nagel 34 ist in dem in Fig. 3 dargestellten Ausführungsbeispiel entlang seiner Außenfläche 36, die in Kontakt mit dem Knochengewebe 38 steht, modifiziert. Dies ist insbesondere vorteilhaft bei einem Bruch 40, dessen Bruchlinie in Höhe des Schaftes des intramedullären Nagels verläuft. Durch die modifizierte Oberfläche 36 kann eine Schädigung des Knochengewebes 38 wirkungsvoll vermindert bzw. verhindert werden.

Fig. 4 zeigt eine schematische Ansicht eines Fixateur Externe. Der Fixateur Externe besitzt eine Klemme 50, mit zwei Öffnungen zur Aufnahme eines Knochen-Pins 52 und einer Konstruktionsstange 54. Die Klemme ist entlang der durch den Doppelpfeil A gekennzeichneten Richtung drehbar. Knochen-Pin 52 und Konstruktionsstange 54 sind jeweils sowohl in Längsrichtung beweglich als auch um ihre Längsachse drehbar in der Klemme 50 angeordnet. Durch Anziehen der Klemme mit Hilfe der Schraube 56 wird die Position von Knochen-Pin 53 und Konstruktionsstange 54 festgelegt. Um eine möglichst große Reibkraft zwischen den Elementen zu erzielen und damit eine erhöhte Sicherheit gegen ein Durchrutschen der Konstruktionsstange bzw. des Knochen-Pins durch die Klemme zu erzielen, sind Klemme 50, Knochen-Pin 52 und Konstruktionsstange 54 in ihren Berührbereichen mit einer modifizierten Oberfläche versehen. Ebenfalls könnten Klemme, Knochen-Pin und Konstruktionsstange auch jeweils für sich mit einer modifizierten Oberfläche versehen sein. Die modifizierte Oberfläche erhöht die Reibung, so daß die Klemmkraft der Klemme 50 vergrößert ist.

## Patentansprüche

1. Osteosynthesehilfsmittel aus einer Stahl-, Cobalt- und/oder Titanlegierung, **dadurch gekennzeichnet, daß** in einem Abschnitt, der im eingesetzten Zustand in Berührung mit Knochengewebe oder mit Metall steht, die Oberfläche modifiziert ist derart, daß Korrosion und Abrieb verringert bzw. verhindert werden.

2. Osteosynthesehilfsmittel nach Anspruch 1, **dadurch gekennzeichnet, daß** der Abschnitt mit Hilfe einer Beschichtung modifiziert ist.

3. Osteosynthesehilfsmittel nach Anspruch 2, **dadurch gekennzeichnet, daß** die Beschichtung durch PVD (physical vapour deposition) oder CVD (chemical vapour deposition) aufgebracht ist.

4. Osteosynthesehilfsmittel nach Anspruch 2 oder 3, **dadurch gekennzeichnet, daß** die Beschichtung als DLC-Schicht (Diamond like carbon) aufgebracht ist.

5. Osteosynthesehilfsmittel nach Anspruch 4, **dadurch gekennzeichnet, daß** die DLC-Schicht den Typ MeC:CH besitzt, bevorzugt mit den Bestandteilen Tantal, Kohlenstoff und Wasserstoff (TaC/CH).

6. Osteosynthesehilfsmittel nach einem der Ansprüche 1 bis 5, **dadurch gekennzeichnet, daß** der Abschnitt durch ein Diffüsionsverfahren gehärtet ist.

7. Osteosynthesehilfsmittel nach Anspruch 6, **dadurch gekennzeichnet, daß** das Diffusionsverfahren bei einer Titanlegierung Stickstoff oder Sauerstoff und bei einer Stahllegierung Kohlenstoff in den Abschnitt einträgt.

8. Osteosynthesehilfsmittel nach einem der Ansprüche 1 bis 7, **dadurch gekennzeichnet, daß** der Abschnitt durch Oberflächenhärtung und -beschichtung mit Hilfe eines Anodisierverfahrens modifiziert ist.

9. Osteosynthesehilfsmittel nach Anspruch 8, **dadurch gekennzeichnet, daß** das Anodisierverfahren eine Anodisation von Typ II oder vom Typ III durch elektrolytische Behandlung erzielt.

10. Osteosynthesehilfsmittel nach einem der Ansprüche 1 bis 9, **dadurch gekennzeichnet, daß** der Abschnitt durch spanende oder spanlose, formgebende Bearbeitungsverfahren modifiziert ist.

11. Osteosynthesehilfsmittel nach Anspruch 10, **dadurch gekennzeichnet, daß** das Bearbeitungsverfahren den Arbeitsschritt des Glas- und/oder Sandstrahlens aufweist.

12. Osteosynthesehilfsmittel nach Anspruch 10 oder 11, **dadurch gekennzeichnet, daß** das Bearbeitungsverfahren den Arbeitsschritt des Schleifens, insbesondere des Gleitschleifens, aufweist.

13. Osteosynthesehilfsmittel nach Anspruch 11 oder 12, **dadurch gekennzeichnet, daß** das Bearbeitungsverfahren den Arbeitsschritt des mechanischen Polierens und/oder des Elektropolieren aufweist.

14. Osteosynthesehilfsmittel nach einem der Ansprüche 1 bis 13, **dadurch gekennzeichnet, daß** als Osteosynthesehilfsmittel ein intramedullärer Nagel und eine oder mehrere Verriegelungsschrauben vorgesehen sind, die in ihrem jeweiligen Kontaktbereich eine modifizierte Oberfläche aufweisen.

15. Osteosynthesehilfsmittel nach einem der Ansprüche 1 bis 14, **dadurch gekennzeichnet, daß** als Osteosynthesehilfsmittel ein intramedullärer Nagel und eine Schenkelhalsschraube vorgesehen sind, die in ihrem jeweiligen Kontaktbereich die modifizierte Oberfläche aufweisen.

16. Osteosynthesehilfsmittel nach einem der Ansprüche 1 bis 15, **dadurch gekennzeichnet, daß** als Osteosynthesehilfsmittel ein intramedullärer Nagel vorgesehen ist, der im Kontaktbereich zu dem Knochengewebe eine modifizierte Oberfläche aufweist.

17. Osteosynthesehilfsmittel nach einem der Ansprüche 1 bis 13, **dadurch gekennzeichnet, daß** als Osteosynthesehilfsmittel eine Hüftplatte und eine Schenkelhalsschraube vorgesehen sind, die in ihrem jeweiligen Kontaktbereich eine modifizierte Oberfläche aufweisen.

18. Osteosynthesehilfsmittel nach einem der Ansprüche 1 bis 13 oder nach Anspruch 17, **dadurch gekennzeichnet, daß** als Osteosynthesehilfsmittel eine Hüftplatte und eine oder mehrere Verriegelungsschrauben vorgesehen sind, die in ihren jeweiligen Kontaktbereichen eine modifizierte Oberfläche aufweisen.

19. Osteosynthesehilfsmittel nach Anspruch 17 oder 18, **dadurch gekennzeichnet, daß** als Osteosynthesehilfsmittel eine Hüftplatte vorgesehen ist, die im Kontaktbereich mit dem Knochen eine modifizierte Oberfläche aufweist.

20. Osteosynthesehilfsmittel aus einer Stahl- und/oder Titanlegierung, **dadurch gekennzeichnet, daß** als Osteosynthesehilfsmittel einen Fixateur Externe mit mindestens einem Knochen-Pin, einer Konstruktionsstange und einer Klemmvorrichtung vorgesehen ist, **dadurch gekennzeichnet, daß** die Klemmeinrichtung und/oder der Knochen-Pin sowie die Konstruktionsstange in ihren jeweiligen Kontaktbereichen eine modifizierte Oberfläche aufweist derart, daß die Reibung zwischen Klemmeinrichtung und Knochen-Pin sowie Konstruktionsstange erhöht ist.
